# EUROPÄISCHE PATENTANMELDUNG

(11) **EP 0 821 919 A2**
(43) Veröffentlichungstag der Anmeldung: **04.02.1998**
(21) Anmeldenummer: 97112975.4
(22) Anmeldetag: 29.07.1997
(51) Int. Cl.: A61F 2/00

(54) **Harnröhrenverschluss**

(30) Priorität: 29.07.1996 DE 19630516; 13.11.1996 DE 19646874
(71) Anmelder: VIA LOG Medikalprodukte GmbH Kosmetik - Medien, D-75378 Bad Liebenzell (DE)
(72) Erfinder: Hutzler, Martin, D-75378 Bad Liebenzell (DE)
(74) Vertreter: KOHLER SCHMID + PARTNER

(57) **Zusammenfassung**

Ein Harnröhrenverschluß 10 ist aus einem stabförmigen Körper 13 und aus einer Platte 11 gebildet. Der stabförmige Körper 13 und die Platte 11 sind aus einem weichen, biegsamen Kunststoffmaterial gefertigt. Die Platte 11 endet radial gesehen in einer verjüngten Randzone 15 mit einem Rand 16. In der Platte 11 ist ein Hohlraum 17 ausgebildet, und der stabförmige Körper 13 weist ein Lumen 18 auf, in das ein Stabilisator 20 einführbar ist. Der Harnröhrenverschluß 10 wird über die gesamte Länge der Harnröhre bis in die Blase vorgeschoben und die Randzone 15 mit dem Rand 16 liegen flüssigkeitsdichtend an der Vulva an. Im Hohlraum 17 kann Urinausfluß zurückgehalten werden, sofern dieser über die Abdichtung durch den stabförmigen Körper 13 nicht vollkommen verhindert werden kann.

## Beschreibung

Die Erfindung betrifft einen Harnröhrenverschluß und ein Verfahren zur Handhabung des Verschlusses, bestehend aus einer gummielastischen Platte, an deren körperzugewandter Fläche ein gummielastischer stabförmiger Körper ausgebildet ist, der im Durchmesser der Harnröhre angepaßt ist und in einer geschlossenen Vollkörperspitze endet.

Ein derartiger Harnröhrenverschluß ist durch die dänische Schutzrechtsanmeldung 84 70 49 A bekanntgeworden.

Der funktionsgerechte Blasenverschluß in Ruhe wird gewährleistet durch zirkuläre glatte Muskelfasern im Bereich der Urethra oberhalb des Diaphragma urogenitale und durch das Venengeflecht unter dem Epithel der Urethra. Für den willkürlichen Verschluß sorgt die quergestreifte Muskulatur zusammen mit den schlingenförmigen Muskelzügen des Diaphragma urogenitale. Bei Frauen kann dieser Blasenverschluß mehr oder weniger stark insuffizient sein. Überdehnungen der Beckenmuskulatur und/oder Läsionen unter der Geburt sowie die spätere Senkung der Vagina und des Uterus können eine Streßinkontinenz auslösen, die sich dadurch bemerkbar macht, daß unter körperlicher Belastung der Harnröhrenverschluß ungenügend ist. Schon beim Lachen, Husten oder Niesen kann es zu einem unkontrollierten Urinabgang kommen.

Der bekannte Harnröhrenverschluß weist eine elastische Platte mit einem daran angeformten Stab auf, der längs des Stabes mindestens eine Verdickung aufweist. Der Stab ist durch eine Einführhilfe versteift, die in ein Lumen des Stabes eingeführt ist. Zum Verschluß der Harnröhre wird dieser durch die Einführhilfe versteifte Stab in die Harnröhre eingeführt. Bei bestimmungsgemäßer Plazierung endet der Stab außerhalb der Blase.

Es hat sich nunmehr gezeigt, daß trotz eines an sich schon guten Verschlusses der Harnröhre weiterhin Urin durch die Harnröhre austreten kann, wenn die am Stab vorgesehenen Verdickungen bei Bewegungen des menschlichen Körpers den Querschnitt der Harnröhre nicht vollkommen verschließen können.

Aufgabe der Erfindung ist es deshalb, eine Vorrichtung zu entwickeln und ein Verfahren zur Handhabung dieser Vorrichtung vorzustellen, die eine vorliegende Stressinkontinenz verbessert unterbindet.

Die Aufgabe bezüglich der Vorrichtung wird dadurch gelöst, daß der stabförmige Körper länger als die Harnröhre ist und sich die Platte zumindest in einer Randzone zum Rand hin verjüngt.

Der erfindungsgemäße Harnröhrenverschluß hat damit den wesentlichen Vorteil, daß er über den eingeführten stabförmigen Körper die gesamte axiale Länge der Harnröhre verschließt und damit nicht nur am Diaphragma urogenitale, sondern auch am M.Sphincter vesicae internus anliegt und diesen zusätzlich künstlich verschließt. Die in einer Randzone verjüngte Platte ist derart ausgebildet, daß sie flüssigkeitsdicht an der Vulva anliegt und somit zusätzlich das unbeabsichtigte Austreten von Urin aus der Harnröhre verhindert. Mit dem erfindungsgemäßen Harnröhrenverschluß wird eine zweifache Sicherheit erreicht, die darin besteht, daß ein durch den stabförmigen Körper nicht vollkommen verhinderter Urinabgang von der an der Vulva flüssigkeitsdicht anliegenden Platte verhindert wird. Sowohl die Platte als auch der stabförmige Körper sind aus einem weichen, biegsamen gewebeverträglichen Kunststoff hergestellt, der sich den menschlichen Körperbewegungen weitgehend anpaßt. Die Oberfläche des stabförmigen Körpers ist an allen Übergängen zur Spitze, an der Spitze und zur Platte mit Rundungen versehen, die sich an den Harnröhrenverlauf anpassen bzw. an den Bereich um die Harnröhre angepaßt sind. Dadurch wird auch ein hoher Tragekomfort ermöglicht.

In weiterer Ausgestaltung der Erfindung ist der stabförmige Körper 30 mm bis 50 mm lang und weist Durchmesser im Charrière-Bereich von 6 bis 16 auf. Mit diesen unterschiedlichen Ausführungsformen eines erfindungsgemäßen Harnröhrenverschlusses werden unterschiedliche Längen von Harnröhren abgedeckt, und es ist sichergestellt, daß bei richtiger Auswahl eines erfindungsgemäßen Harnröhrenverschlusses der stabförmige Körper immer vollkommen die Harnröhre durchdringt und mit seiner freien Spitze geringfügig in die Blase hineinragt.

In einer Weiterbildung der Erfindung ist der Rand als Dichtungskante ausgebildet. Der sehr dünn auslaufende Rand legt sich membranartig an die Vulva im Bereich der Harnröhre und dichtet diesen Gewebebereich ab. Damit kann über die Dichtungskante hinaus kein Urinabgang stattfinden. In bevorzugter Ausgestaltung ist dabei noch die Randzone als Dichtungsfläche ausgebildet. Somit liegt nicht nur eine Abdichtung über die Dichtungskante, sondern auch über einen verbreiterten Flächenabschnitt vor. Die Dichtungszonen über den Rand und über die flächenhafte Erstreckung in der Randzone verhindern zuverlässig, daß Urin über diese Flächenbereiche hinaus austreten kann.

In weiterer bevorzugter Ausgestaltung der Erfindung weist die Platte zwischen dem Rand bzw. zwischen der Randzone und dem stabförmigen Körper einen Hohlraum auf, der um den stabförmigen Körper umlaufend ausgebildet ist.

Dies hat den Vorteil, daß sofern Kleinstmengen von Urin dennoch den Harnröhrenverschluß überwinden sollten, dieser Urin im Hohlraum aufgefangen gehalten werden kann. Der Hohlraum dient als Reservoir und kann mehrere Urintropfen aufnehmen. Der Hohlraum selbst ist über eine Flächenabdichtung in der Randzone bzw. über die Dichtungskante am Rand abgedichtet, so daß der im Hohlraum aufgefangene Urin erst beim Entfernen des Harnröhrenverschlusses aus dem Hohlraum entleert werden kann.

In weiterer Ausgestaltung der Erfindung weist der stabförmige Körper ein Lumen auf, das einerseits am Übergang zur Vollkörperspitze verschlossen und andererseits an der Platte offen endet.

Dies hat den Vorteil, daß der stabförmige Körper im eingeführten Zustand des Harnröhrenverschlusses durch das Lumen noch biegsamer und beweglicher in der Harnröhre plaziert ist. Bei Bewegungen des menschlichen Körpers kann bei Muskelanspannungen die Außenoberfläche des stabförmigen Körpers reversibel membranartig nachgeben und sich den Verformungen in der Harnröhre bestmöglich anpassen und damit auch die Dichtungsfunktion verbessern.

Das Lumen ist an der Platte offen, so daß in das Lumen über die gesamte axiale Länge des Lumens ein Stabilisator einführbar ist, der aus einem formstabileren Material als der stabförmige Körper hergestellt ist. Mit dem Stabilisator kann der stabförmige Körper versteift werden und somit richtungsstabiler in die Harnröhre verbessert eingeführt werden. Der Stabilisator kann ein Führungsdraht sein, über den auch die Manövrierbarkeit des stabförmigen Körpers erhöht wird. Am Ende des Stabilisators ist ein Griffteil ausgebildet, über den der Harnröhrenverschluß gehalten werden kann. Damit ist gewährleistet, daß beim Einführen des Harnröhrenverschlusses der Harnröhrenverschluß selbst an Stellen, die mit der Harnröhre in Berührung kommen, nicht angefaßt werden muß. Nachdem der Harnröhrenverschluß plaziert ist, wird der Stabilisator entfernt, damit die Beweglichkeit des Harnröhrenverschlusses erhöht wird und über das Lumen eine zusätzliche Beweglichkeit der Materialflächen am stabförmigen Körper ermöglicht wird.

In weiterer bevorzugter Ausgestaltung der Erfindung ist dem Harnröhrenverschluß ein mit Gleitmittel und/oder einem infektionshemmenden Medikament gefülltes Behältnis zugeordnet.

Dies hat den Vorteil, daß der stabförmige Körper gleitmittelbehaftet erleichtert in die Harnröhre eingeführt werden kann. Ist dem Gleitmittel noch ein infektionshemmendes Medikament beigefügt, so wird die Sicherheit beim Tragen eines erfindungsgemäßen Harnröhrenverschlusses noch weiter erhöht, und möglicherweise dabei auftretende Infektionen werden verhindert.

Die Aufgabe wird auch durch ein Verfahren zur Handhabung des erfindungsgemäßen Harnröhrenverschlusses dadurch gelöst, daß der sterile Harnröhrenverschluß mit dem durch den Stabilisator versteiften stabförmigen Körper in ein Gleitmittel und/oder ein Medikament eingetaucht wird und daß anschließend der benetzte stabförmige Körper soweit in die Harnröhre eingeführt wird, daß der Schaft mit dem freien Ende der Vollkörperspitze in die Blase vorsteht und die Dichtungskante und/oder die Dichtungsfläche an der Vulva im Breich der Harnröhre anliegt.

Mit dem erfindungsgemäßen Verfahren wird eine schonende Plazierung und eine gute Abdichtung der Harnröhre an der Vulva erreicht. Das Gleitmittel erleichtert das Einführen des stabförmigen Körpers. Nachdem der stabförmige Körper in seine bestimmungsgemäße Endlage in die Harnröhre vorgeschoben wurde, wird der Stabilisator entfernt. Die Dichtungskanten und/oder die Dichtungsflächen liegen flüssigkeitsdicht an der Vulva an, so daß der Harnröhrenverschluß lagefixiert ist und nicht nach innen in die Harnröhre weiter vordringen kann. Der erfindungsgemäße Harnröhrenverschluß paßt sich menschlichen Körperbewegungen an und schmiegt sich dichtend an die Innenoberfläche der Harnröhre.

In bevorzugter Handhabung wird der stabförmige Körper mit der Vollkörperspitze nach der Benetzung des stabförmigen Körpers mit dem Gleitmittel und/oder dem Medikament senkrecht nach oben gehalten.

Dies hat den Vorteil, daß der stabförmige Körper, bevor er in die Harnröhre eingeführt wird, vollkommen oberflächenbenetzt ist, und zwar über seine gesamte axiale Länge. Bei einer derartigen Handhabung könnte auch nur die Vollkörperspitze mit einem fließfähigen Gleitmittel bzw. einem Medikament benetzt werden und nach dem Auftragen des Medikaments bzw. des Gleitmittels im Spitzenbereich strömt das Medikament entlang des stabförmigen Körpers nach unten zur Platte. Ist der Gleitmittelfluß an der Platte angekommen, so kann der Harnröhrenverschluß plaziert werden.

Bevorzugt wird auch beim Einführen des Harnröhrenverschlusses der Hohlraum an der Platte reversibel deformiert.

Dies hat den Vorteil, daß sich nach plaziertem Harnröhrenverschluß die Dichtungskante bzw. die Dichtungsfläche an der Vulva festsaugen kann. Somit wird der Harnröhrenverschluß auch bei stärkerer körperlicher Tätigkeit immer in seiner vorbestimmten Lage gehalten und die Dichtungsfunktion im Bereich der Platte wird nochmals verbessert. Ist die Platte mit einem Reservoir versehen, so ist durch die unter geringfügigem Druck anliegenden Dichtungsflächen bzw. Dichtungskante gewährleistet, daß im Reservoir aufgefangener Urin nicht über die Dichtungskante nach außen treten kann.

In weiteren Ausgestaltungen der Erfindung kann der erfindungsgemäße Harnröhrenverschluß mit einem axial verschiebbaren bzw. verformbaren Abschnitt am stabförmigen Körper ausgebildet sein. Dieser verschiebbare bzw. verformbare Abschnitt ermöglicht im plazierten Zustand des Harnröhrenverschlusses eine zusätzliche Abdichtung des stabförmigen Körpers in der Blase. Er bildet gleichzeitig eine Lagesicherung des Harnröhrenverschlusses und verhindert zuverlässig eine ungewollte axiale Verschiebung des Harnröhrenverschlusses in der Harnröhre. Die Wirkungsweisen und der Aufbau des verschiebbaren bzw. verformbaren Abschnittes sind in den nachfolgenden Figuren erläutert.

Weitere Vorteile und Einzelheiten ergeben sich aus der beigefügten Zeichnung und der Figurenbeschreibung. Die in der Beschreibung aufgeführten Merkmale können in beliebigen Kombinationen miteinander verwendet werden. Ausführungsbeispeile eines erfindungsgemäßen Harnröhrenverschlusses sind in den nachfolgenden Figuren beschrieben.

Es zeigt:
- Fig. 1: einen erfindungsgemäßen Harnröhrenverschluß, teilweise im Querschnitt;
- Fig. 2: einen erfingungsgemäßen Harnröhrenverschluß, wie er in der Harnröhre mit einem Stabilisator plaziert ist;
- Fig. 3: einen erfindungsgemäßen Harnröhrenverschluß mit entferntem Stabilisator in bestimmungsgemäßer Endlage in der Harnröhre;
- Fig. 4: einen erfindungsgemäßen Harnröhrenverschluß in Seitenansicht;
- Fig. 5: eine weitere Ausführungsform eines erfindungsgemäßen Harnröhrenverschlusses in Seitenansicht;
- Fig. 6: eine Draufsicht auf eine Dichtungskante und eine Dichtungsfläche sowie auf einen Hohlraum eines Harnröhrenverschlusses mit kreisförmig ausgebildeter Platte;
- Fig. 7: eine Draufsicht auf eine Dichtungskante und eine Dichtungsfläche einer oval ausgebildeten Platte eines erfindungsgemäßen Harnröhrenverschlusses;
- Fig. 8: einen erfindungsgemäßen Harnröhrenverschluß mit einem axial verschiebbaren Abschnitt am stabförmigen Körper im ausgestreckten Zustand;
- Fig. 9: einen erfindungsgemäßen Harnröhrenverschluß im deformierten Zustand des Abschnittes gemäß Fig. 8;
- Fig. 10: einen erfindungsgemäßen Harnröhrenverschluß im gedehnten Zustand des stabförmigen Körpers und im eingeführten Zustand in die Harnröhre;
- Fig. 11: einen erfindungsgemäßen Harnröhrenverschluß gemäß Fig. 10 im dehnungsfreien Zustand unter Ausbildung eines Ballons am Blasenboden.

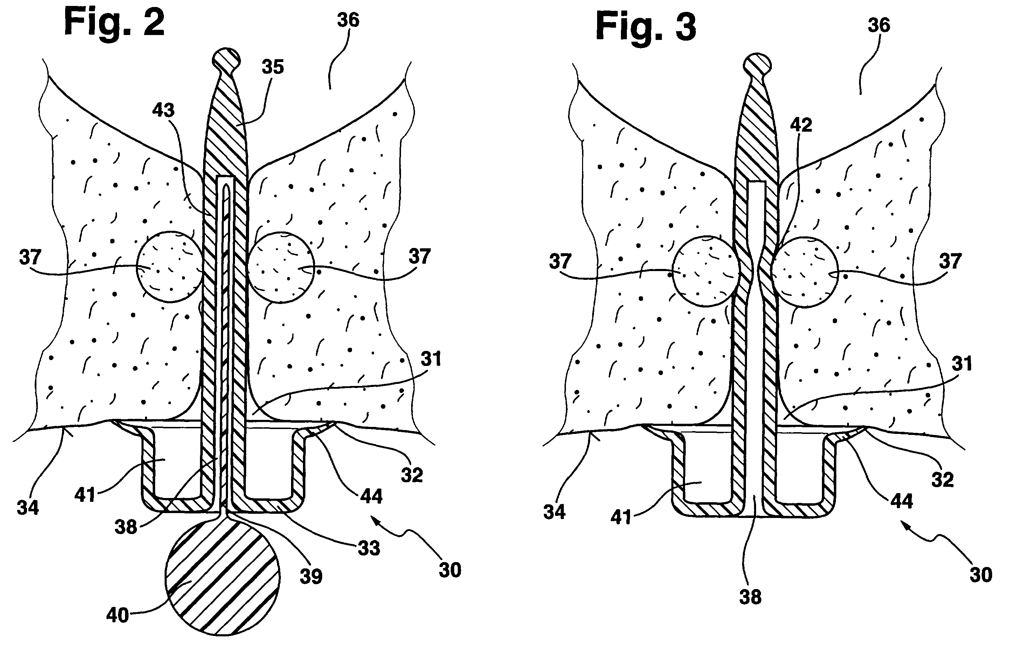

Die in den Figuren dargestellten Ausführungsformen sind stark schematisiert dargestellt und sind nicht maßstäblich zu verstehen. In den Fig. 2 und 3 ist die Blase und die Harnröhre stark vereinfacht dargestellt und das Diaphragma urogenitale ist durch jeweils zwei Kreise angedeutet.

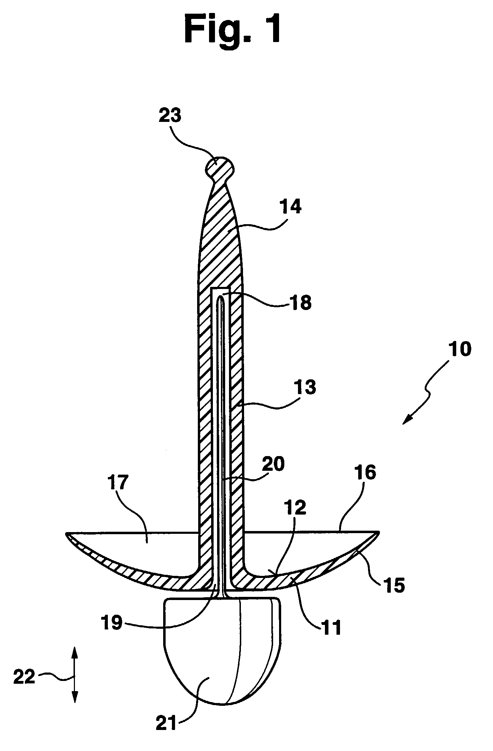

Fig. 1 zeigt mit 10 einen Harnröhrenverschluß, der eine Platte 11 aufweist, die an einer körperzugewandten Fläche 12 einen stabförmigen Körper 13 angeformt hat. Der stabförmige Körper 13 endet in einer Vollkörperspitze 14. An der Platte 11 ist eine sehr bewegliche Randzone 15 ausgebildet, die in einem Rand 16 endet.

Sowohl die Platte 11 wie auch der stabförmige Körper 13 sind aus einem sehr weichen, biegsamen und gewebeverträglichen Kunststoff gefertigt, der an seiner Außenoberfläche eine hohe Oberflächengüte aufweist. Der Rand 16 ist als Dichtkante ausgebildet und die Randzone 15 als Dichtungsfläche. Zwischen der Randzone 15 und dem stabförmigen Körper 13 ist ein Hohlraum 17 ausgebildet. Der Hohlraum 17 entsteht durch eine gewölbte, konkave Fläche, die sich vom Rand 16 zum stabförmigen Körper 13 erstreckt.

Der stabförmige Körper 13 weist über seine axiale Länge bis zur Vollkörperspitze 14 ein Lumen 18 auf, das in einer Öffnung 19 an der Unterseite der Platte 11 endet. Über die Öffnung 19 ist ein Stabilisator 20 in das Lumen 18 einführbar. Dabei kann der Stabilisator 20 an einem Griffteil 21 gehalten werden. In Pfeilrichtung 22 ist der Stabilisator 20 innerhalb des Lumens 18 verschiebbar. Der Stabilisator 20 kann ein Mandrin sein, der den an sich sehr beweglichen und biegsamen stabförmigen Körper 13 ausrichtet und versteift. Über den Stabilisator 20 kann der stabförmige Körper 13 richtungsvorgebend gesteuert werden.

Der in der Figur gezeigte stabförmige Körper 13 endet in einer kugelförmigen Spitze 23, die über einen halsförmigen Abschnitt an die Vollkörperspitze 14 angeformt ist. In der ringförmigen Ausnehmung zwischen der kugelförmigen Spitze 23 und der konisch verlaufenden Vollkörperspitze 14 kann ein Gleitmitteldepot vorgesehen sein, das sich beim Verschieben des stabförmigen Körpers 13 in der Harnröhre längs des stabförmigen Körpers 13 in Richtung Platte 11 verteilt und damit den Reibungswiderstand zwischen der Innenoberfläche der Harnröhre und der Außenoberfläche des stabförmigen Körpers 13 minimiert.

Fig. 2 zeigt einen Harnröhrenverschluß 30, der in eine Harnröhre 31 eingesetzt ist. Ein Rand 32 einer Platte 33 liegt flüssigkeitsdicht an der Vulva 34 an. Eine Vollkörperspitze 35 ragt in die Blase 36, so daß gewährleistet ist, daß mit dem Harnröhrenverschluß 30 die gesamte Länge der Harnröhre 31 verschlossen ist.

Mit 37 ist stark vereinfacht das Diaphragma urogenitale angedeutet, dem auch der M.Sphincter vesicae externus angehört. Nahe am Blasenboden ist der M.Sphincter vesicae internus ausgebildet, der in der Figur nicht dargestellt ist. Der Harnröhrenverschluß 30 durchdringt bei einer bestimmungsgerechten Plazierung sowohl das Diaphragma urogenitale als auch den M.Sphincter vesicae internus.

Zur Plazierung des Harnröhrenverschlusses 30 ist in ein Lumen 38 ein Stabilisator 39 eingeschoben, der die Richtungsstabilität des Harnröhrenverschlusses 30 erhöht. Über ein Griffteil 40 kann der Stabilisator 39 im Lumen 38 verschoben werden.

Die Platte 33 beinhaltet innerhalb des Randes 32 einen Hohlraum 41, der sich zwischen dem Rand 32 und einer Außenoberfläche 42 eines stabförmigen Körpers 43 erstreckt. Zwischen dem Rand 32 und dem Hohlraum 41 ist noch eine Randzone 44 ausgebildet, die sich in der Regel dichtend an die Vulva 34 anlegt.

Fig. 3 zeigt den Harnröhrenverschluß 30 im eingeführten Zustand in der Harnröhre 31, wobei aus dem Lumen 38 der Stabilisator entfernt wurde. Über den flüssigkeitsdicht an der Vulva 34 anliegenden Rand 32 ist der Harnröhrenverschluß 30 lagefixiert und kann sich nicht unbeabsichtigt verschieben. Der Rand 32 und die Randzone 44 dichten den Hohlraum 41 gegenüber der Vulva 34 ab.

Der stabförmige Körper 43 ragt soweit in die Blase 36, daß er sowohl die Harnröhre im Bereich des M.Sphincter vesicae internus wie auch im Bereich des M.Sphincter vesicae externus künstlich verschließt. In diesen Bereichen des stabförmigen Körpers 43 ist das Lumen 38 ausgebildet, so daß sich der stabförmige Körper 43 bei Kontraktionen im Diaphragma urogenitale membranartig verändern kann. Durch das Lumen 38 kann sich die Kontur der Außenoberfläche 42 verändern und somit zu einer verbesserten Abdichtung in der Harnröhre beitragen. Durch die hohe Flexibilität des stabförmigen Körpers 43 wird auch der Tragekomfort erhöht und der Harnröhrenverschluß 30 wird wesentlich weniger als Fremdkörper empfunden.

Über den stabförmigen Körper in der Harnröhre 31 und über die Dichtungskante, die durch den Rand 32 gebildet ist, findet eine zweifache Abdichtung gegenüber einem unwillkürlichen Urinabgang statt.

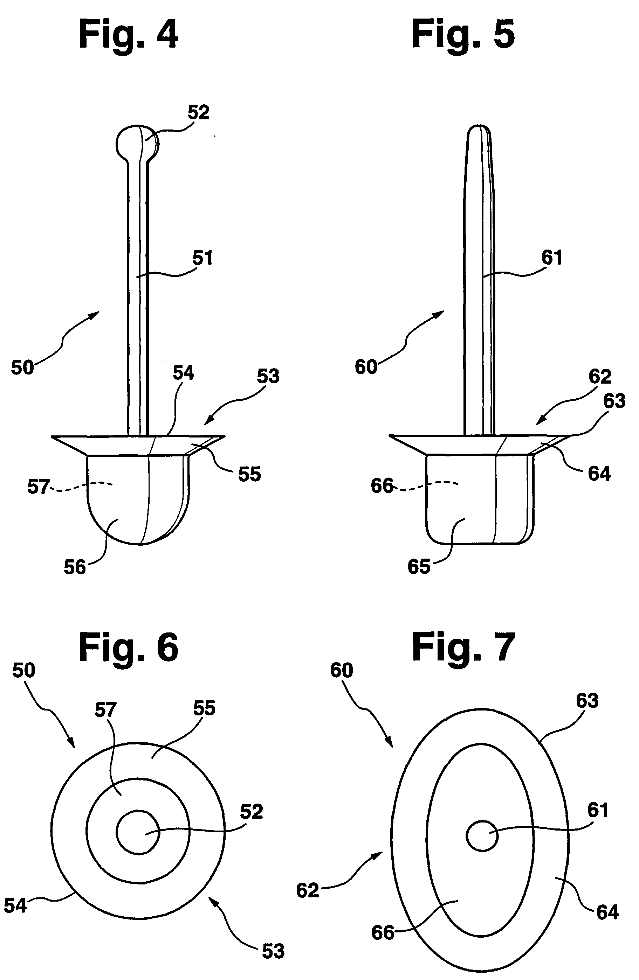

Fig. 4 zeigt ein weiteres Ausführungsbeispiel eines Harnröhrenverschlusses 50, der einen stabförmigen Körper 51 aufweist, der an seinem freien Ende in einer als Kugel 52 ausgeformten Spitze endet. Am anderen Ende des stabförmigen Körpers 51 ist eine Platte 53 ausgebildet, aus der zentral der stabförmige Körper 51 vorsteht. Die Platte 53 selbst setzt sich aus einem Rand 54, einer Randzone 55 und aus einem Reservoir 56 zusammen. Das Reservoir 56 beinhaltet einen Hohlraum 57, der sowohl die Funktion eines Flüssigkeitsspeichers wie auch eines Gleitmitteldepots übernehmen kann. In den stabförmigen Körper 51 kann bei Bedarf ein Stabilisator 20 eingeführt werden.

Fig. 5 zeigt ein weiteres Ausführungsbeispiel eines Harnröhrenverschlusses 60, der einen stabförmigen Körper 61 aufweist, der an seinem freien Ende in einer konisch verjüngten Spitze endet. Die am stabförmigen Körper 61 ausgebildete Platte 62 setzt sich aus einem Rand 63, einer Randzone 64 und aus einem Reservoir 65 zusammen. Das Reservoir 65 begrenzt einen Hohlraum 66.

Fig. 6 zeigt in Draufsicht den Harnröhrenverschluß 50 mit der Platte 53, die kreisförmig ausgebildet ist, und aus der zentrisch der stabförmige Körper vorsteht, der in einer als Kugel 52 ausgeformten Spitze endet. An den Rand 54 schließt sich die Randzone 55 und daran der Hohlraum 57 an.

Fig. 7 zeigt in Draufsicht den Harnröhrenverschluß 60 mit einer oval ausgebildeten Platte 62, die in dem Rand 63 endet und an den sich nach innen die Randzone 64 und daran der Hohlraum 66 anschließt. Mittig aus dem Hohlraum 66 ragt der stabförmige Körper 61, der am Boden des Hohlraumes 56 an der Platte 62 befestigt ist.

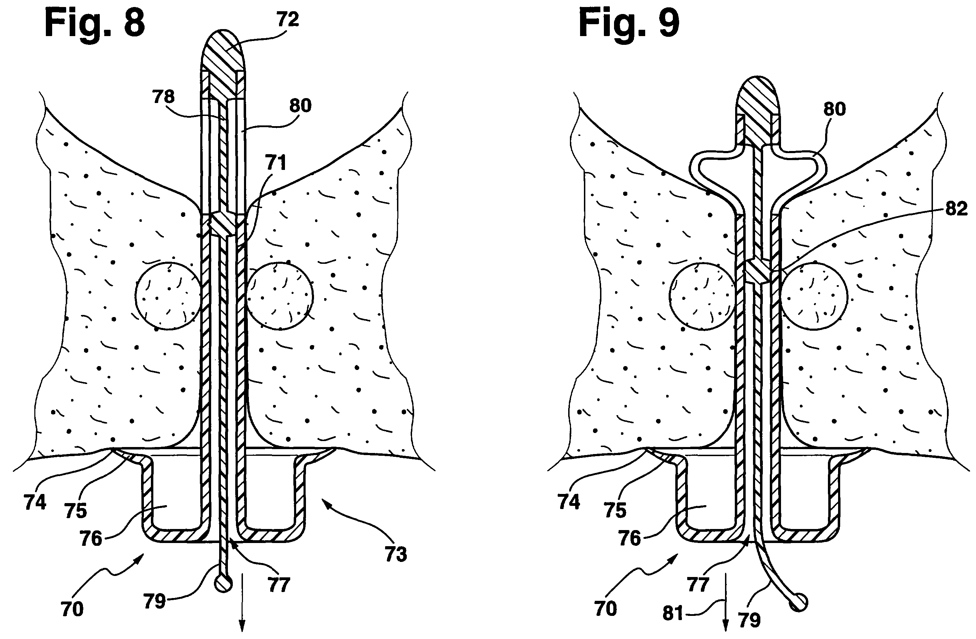

Fig. 8 zeigt einen Harnröhrenverschluß 70, wie er in der Harnröhre plaziert ist. Ein stabförmiger Körper 71 ist durch die Harnröhre hindurchgeführt und das freie Ende des stabförmigen Körpers 71 ragt in die Blase. Das freie Ende des stabförmigen Körpers 71 endet in der Blase in einer abgerundeten Spitze 72. Das dazu distale Ende des Harnröhrenverschlusses 70 endet in einer Platte 73, die an der Vulva über einen Rand 74 und eine Randzone 75 anliegt. Die Platte 73 mit dem Rand 74 und der Randzone 75 weist zusätzlich noch ein Reservoir 76 auf.

Der stabförmige Körper 71 ist mit einem Lumen 77 ausgebildet, in dem ein Stabilisator 78 geführt gehalten ist. Der Stabilisator 78 steht mit einem Endstück 79 über die Platte 73 vor. Der Stabilisator 78 kann im Endbereich oder abschnittsweise als reißfester Faden oder dergleichen ausgebildet sein. Längs des stabförmigen Körpers 71 ist im Bereich der Blase ein axial beweglicher Abschnitt 80 ausgebildet. Der Stabilisator 78 ist über das proximale Ende fest mit der abgerundeten Spitze 72 verbunden. Zieht man das Endstück 79 in Pfeilrichtung 81, so kann sich der Abschnitt 80 reversibel deformieren. Der Abschnitt 80 ist bevorzugt aus einem gummielastischen Material gefertigt. Im Ausführungsbeispiel der Figur 8 ist der gummielastische Abschnitt 80 aus Streifen gebildet, die über Schlitze voneinander beabstandet sind. Es versteht sich jedoch, daß der gummielastische Abschnitt auch aus einem zylindrischen Hohlkörper gebildet sein kann, der sich im deformierten Zustand radial verdickend auswölbt. Die beispielsweise ballonartige Auswölbung weist eine Größe auf, die wesentlich größer ist als der Durchmesser der Harnröhre. Dies hat den Vorteil, daß der erfindungsgemäße Harnröhrenverschluß 70 nicht nur dichtend über die Platte 73 an der Vulva anliegt, sondern auch einen weiteren dichtenden Abschluß am Blasenübergang zur Harnröhre bildet. Im aktivierten Zustand ist der erfindungsgemäße Harnröhrenverschluß 70 unverrückbar in der Harnröhre plaziert.

Fig. 9 zeigt den erfindungsgemäßen Harnröhrenverschluß 70 im aktivierten Zustand. Das Endstück 79 wurde in Pfeilrichtung 81 gezogen und dabei hat sich der gummielastische Abschnitt 80 verdickend aufgewölbt und sich an die Blaseninnenwandung angelegt. Der Harnröhrenverschluß 70 ist in der gezeigten Darstellung über den Rand 74, die Randzone 75, über den gummielastischen Abschnitt 80 und über einen membranartig nachgiebigen Abschnitt des stabförmigen Körpers 71, der am Diaphragma urogenitale anliegt, abgedichtet und lagestabil fixiert. Am Stabilisator 78 ist noch ein Dichtring 82 ausgebildet, der das Lumen 77 flüssigkeitsdicht verschließt. Ebenfalls sind am Endstück 79 Mittel vorgesehen, die den Stabilisator 78 bezüglich einer axialen Verschiebung arretieren können (in der Figur nicht dargestellt).

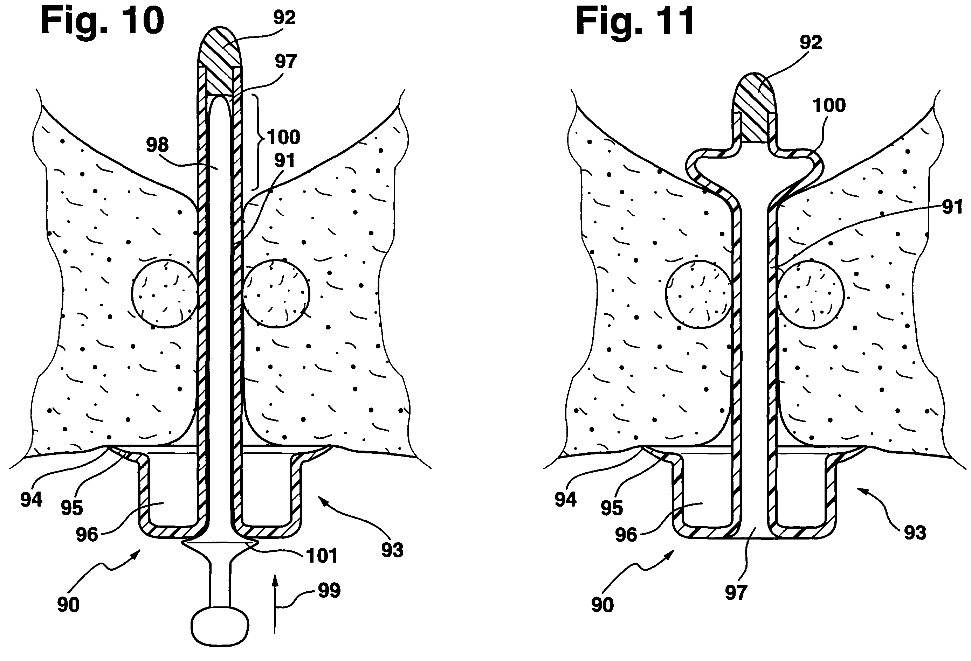

Fig. 10 zeigt einen Harnröhrenverschluß 90 im schon plazierten Zustand, wie er in die Blase ragt. Ein stabförmiger Körper 91 ist durch die Harnröhre bis in die Blase vorgeschoben. Mit einer Spitze 92 ragt der stabförmige Körper 91 in die Blase. Am zur Spitze 92 diametralen Ende ist am stabförmigen Körper 91 eine Platte 93 ausgebildet, die in einem Rand 94 endet. Der Rand 94 ist von einer Randzone 95 umgeben, die sich mit dem Rand 94 an das angrenzende Gewebe dichtend anlegt. In der Platte 93 ist ein Reservoir 96 ausgebildet, das durch die Randzone 95 begrenzt ist. Die Funktion des Reservoirs 96 entspricht den schon in den vorangestellten Figuren beschriebenen Reservoirs.

Im stabförmigen Körper 91 ist ein Lumen 97 zur Aufnahme eines Stabilisators 98 ausgebildet. Der Stabilisator 98 kann in Pfeilrichtung 99 in das Lumen eingeschoben werden. Mit dem Stabilisator 98 kann ein verformbarer Abschnitt 100 des stabförmigen Körpers 91 in axialer Richtung gedehnt werden. Der verformbare Abschnitt 100 ist aus einem elastischen Material gefertigt. Am Stabilisator 98 ist ein Anschlag 101 ausgebildet, der größer ist als das Lumen 97. Der Anschlag 101 verhindert, daß der stabförmige Körper 91 unzulässig stark gedehnt werden kann.

Fig. 11 zeigt den Harnröhrenverschluß 90 im ursprünglichen, dehnungsfreien Zustand des stabförmigen Körpers 91. Der Stabilisator 98 ist entfernt und der stabförmige Körper 91 hat sich auf seine ursprüngliche Form selbsttätig zurückgebildet. Die Spitze 92 hat sich zum Blasenboden hin zurückgezogen und der verformbare Abschnitt 100 hat sich ballonartig über den Blasenboden ausgebreitet. Der ballonartig ausgebildete Abschnitt 100 dichtet den Harnröhrenverschluß 90 zusätzlich gegenüber der Blase ab und fixiert den Harnröhrenverschluß 90 am Blasenboden. Am dazu gegenüberliegenden Ende des Harnröhrenverschlusses 90 fixiert die Platte 93 den stabförmigen Körper 91 am angrenzenden Gewebe. Der Rand 94 mit der Randzone 95 dichtet die Platte 93 flüssigkeitsdicht ab, so daß in das Reservoir 96 eintretende Flüssigkeit im Reservoir 96 so lange gefangen bleibt, bis der Harnröhrenverschluß 90 aus der Harnröhre herausgezogen wird. Das Lumen 97 ist frei, so daß der stabförmige Körper 91 über seine gesamte axiale Länge eine Nachgiebigkeit auf von außen einwirkende Drücke ermöglicht. Durch die radiale Beweglichkeit des stabförmigen Körpers 91 ist es gewährleistet, daß sich die Außenoberfläche des stabförmigen Körpers 91 bestmöglich an die Innenoberfläche der Harnröhre anschmiegt.

Der Stabilisator kann auch derart ausgeführt und auf den stabförmigen Körper abgestimmt sein, daß mit dem Stabilisator der flexible stabförmige Körper und der verformbare Abschnitt so stark gedehnt werden können, daß sich die radiale Erstreckung des stabförmigen Körpers und des verformbaren Abschnittes stark verjüngt. Dies hat den Vorteil, daß der Harnröhrenverschluß unter einem verringerten Widerstand durch die Harnröhre hindurch bis in die Blase vorgeschoben werden kann.

Der erfindungsgemäße Harnröhrenverschluß 10, 30, 50, 60 weist jeweils eine zweistufige Abdichtung der Harnröhre 31 auf, und die stabförmigen Körper 13, 43, 51, 61 sind sowohl in radialer wie auch in axialer Richtung elastisch ausgebildet. Die radiale Membranwirkung ergibt sich aus dem in den stabförmigen Körpern ausgebildeten Lumen, und die axiale Membranwirkung ergibt sich aus der beweglich angeordneten Dichtungskante bzw. Dichtungsfläche der Platten 11, 33, 53, 62. Die Vollkörperspitzen 14, 35, 52 und die konisch verjüngte Spitze des Harnröhrenverschlusses 60 können Depotausnehmungen an der Außenoberfläche der Spitze aufweisen, in denen Gleitmittel eingelagert sein kann. Wird der stabförmige Körper in die Harnröhre eingeführt, so wird das Gleitmittel im Spitzenbereich freigegeben und gleitet entlang des stabförmigen Körpers in Richtung des Hohlraums in der Platte.

Der Harnröhrenverschluß 70, 90 kann ebenfalls die gegenständlichen Ausbildungen der Harnröhrenverschlüsse 10, 30, 50, 60 aufweisen und ist darüberhinaus noch mit einer zusätzlichen Abdichtung bzw. Fixierung versehen.

Ein Harnröhrenverschluß 10 ist aus einem stabförmigen Körper 13 und aus einer Platte 11 gebildet. Der stabförmige Körper 13 und die Platte 11 sind aus einem weichen, biegsamen Kunststoffmaterial gefertigt. Die Platte 11 endet radial gesehen in einer verjüngten Randzone 15 mit einem Rand 16. In der Platte 11 ist ein Hohlraum 17 ausgebildet, und der stabförmige Körper 13 weist ein Lumen 18 auf, in das ein Stabilisator 20 einführbar ist. Der Harnröhrenverschluß 10 wird über die gesamte Länge der Harnröhre bis in die Blase vorgeschoben und die Randzone 15 mit dem Rand 16 liegen flüssigkeitsdichtend an der Vulva an. Im Hohlraum 17 kann Urinausfluß zurückgehalten werden, sofern dieser über die Abdichtung durch den stabförmigen Körper 13 nicht vollkommen verhindert werden kann.

## Patentansprüche

1. Harnröhrenverschluß, bestehend aus einer gummielastischen Platte (11; 33; 53; 62), an deren körperzugewandter Fläche (12) ein gummielastischer stabförmiger Körper (13; 43; 51; 61) ausgebildet ist, der im Durchmesser der Harnröhre (31) angepaßt ist, und in einer geschlossenen Vollkörperspitze (14; 35; 52) endet,
dadurch gekennzeichnet,
daß der stabförmige Körper (13; 43; 51; 61) länger als die Harnröhre (31) ist, und sich die Platte (11; 33; 53; 62) zumindest in einer Randzone (15; 44; 55; 64) zum Rand (16; 32; 54; 63) hin verjüngt.

2. Harnröhrenverschluß nach Anspruch 1, dadurch gekennzeichnet, daß der stabförmige Körper (13; 43; 51; 61) 30 mm bis 50 mm lang ist.

3. Harnröhrenverschluß nach Anspruch 1 oder 2, dadurch gekennzeichnet, daß der Rand (16; 32; 54; 63) als Dichtungskante ausgebildet ist.

4. Harnröhrenverschluß nach einem der Ansprüche 1 bis 3, dadurch gekennzeichnet, daß die Randzone (15; 44; 55; 64) als Dichtungsfläche ausgebildet ist.

5. Harnröhrenverschluß nach einem der Ansprüche 1 bis 4, dadurch gekennzeichnet, daß die Platte (11; 33; 53; 62) zwischen dem Rand (16; 32; 54; 63) bzw. zwischen der Randzone (15; 44; 55; 64) und dem stabförmigen Körper (13; 43; 51; 61) einen Hohlraum (17; 41; 57; 66) aufweist, der um den stabförmigen Körper (13; 43; 51; 61) umlaufend ausgebildet ist.

6. Harnröhrenverschluß nach Anspruch 5, dadurch gekennzeichnet, daß der stabförmige Körper (13; 43; 51; 61) ein Lumen (18; 38) aufweist, das einerseits am Übergang zur Vollkörperspitze (14; 35; 52) verschlossen und andererseits an der Platte (11; 33; 53; 62) offen endet.

7. Harnröhrenverschluß nach Anspruch 6, dadurch gekennzeichnet, daß über die gesamte axiale Länge des Lumens (18; 38) ein Stabilisator (20; 39) einführbar ist, der aus einem formstabileren Material als der stabförmige Körper (13; 43) hergestellt ist.

8. Harnröhrenverschluß nach Anspruch 7, dadurch gekennzeichnet, daß der Stabilisator (20; 39) über das offene Ende (19) vorsteht und in einem vergrößerten Griffteil (21; 40) endet.

9. Harnröhrenverschluß nach einem der Ansprüche 1 bis 8, dadurch gekennzeichnet, daß dem Harnröhrenverschluß (10; 30; 50; 60) ein mit Gleitmittel und/oder einem infektionshemmenden Medikament gefülltes Behältnis zugeordnet ist.

10. Verfahren zur Handhabung eines Harnröhrenverschlusses (10; 30; 50; 60) nach einem der Ansprüche 1 bis 9, dadurch gekennzeichnet, daß der sterile Harnröhrenverschluß (10; 30; 50; 60) mit im stabförmigen Körper (13; 43; 51; 61) eingeführtem Stabilisator (20; 39) in ein Gleitmittel und/oder ein Medikament eingetaucht wird und daß anschließend der benetzte stabförmige Körper (13; 43; 51; 61) soweit in die Harnröhre 31 eingeführt wird, daß der stabförmige Körper (13; 43; 51; 61) mit dem freien Ende der Vollkörperspitze (14; 35; 52) in die Blase (36) vorsteht und der Rand (16; 32; 54; 63) und/oder die Randzone (15; 44; 55; 64) an der Vulva im Bereich der Harnröhre (31) dichtend anliegt.

11. Verfahren nach Anspruch 10, dadurch gekennzeichnet, daß nach der Benetzung des stabförmigen Körpers (13; 43; 51; 61) mit dem Gleitmittel und/oder dem Medikament der stabförmige Körper (13; 43; 51; 61) mit der Vollkörperspitze (14; 35; 52) senkrecht nach oben gehalten wird.

12. Verfahren nach Anspruch 10 oder 11, dadurch gekennzeichnet, daß ein Hohlraum (17; 41; 57; 66) an der Platte (11; 33; 53; 62) beim Einführen des Harnröhrenverschlusses (10; 30; 50; 60) in die Harnröhre reversibel deformiert wird.

13. Harnröhrenverschluß nach einem der Ansprüche 1 bis 10, dadurch gekennzeichnet, daß am stabförmigen Körper (71) ein in axialer Richtung verschiebbarer Abschnitt (80) vorgesehen ist, der im ausgestreckten Zustand der radialen Erstreckung des stabförmigen Körpers (71) entspricht und in einem über einen Stabilisator (78) deformierten Zustand gegenüber den an den Abschnitt (80) jeweils in axialer Richtung angrenzenden Bereichen des stabförmigen Körpers (71) in radialer Richtung vergrößert ist.

14. Harnröhrenverschluß nach einem der Ansprüche 1 bis 10, dadurch gekennzeichnet, daß am stabförmigen Körper (91) ein in axialer Richtung elastisch verformbarer Abschnitt (100) vorgesehen ist, der im gedehnten Zustand im wesentlichen der radialen Erstreckung des stabförmigen Körpers (91) in axialer Richtung entspricht und im dehnungsfreien Zustand gegenüber den an den Abschnitt (100) jeweils in axialer Richtung angrenzenden Bereichen des stabförmigen Körpers (91) in radialer Richtung vergrößert ist.

15. Harnröhrenverschluß nach Anspruch 14, dadurch gekennzeichnet, daß im stabförmigen Körper (91) ein Lumen (97) zur Aufnahme eines Stabilisators (98) ausgebildet ist, der die Deformation mindestens des verformbaren Abschnitts (100) auf eine radiale Erstreckung erlaubt, die der übrigen radialen Erstreckung des stabförmigen Körpers (91) entspricht.

16. Harnröhrenverschluß nach Anspruch 15, dadurch gekennzeichnet, daß durch den Stabilisator (98) eine Verjüngung des gesamten stabförmigen Körpers (91) einschließlich des verformbaren Abschnitts (100) ermöglicht ist, die kleiner als die radiale Erstreckung in an den Abschnitt (100) angrenzenden Bereichen des stabförmigen Körpers 91 im dehnungsfreien Zustand ist.

17. Harnröhrenverschluß nach einem der Ansprüche 14 bis 16, dadurch gekennzeichnet, daß der verformbare Abschnitt (100) im dehnungsfreien Zustand einen Ballon bildet, der gegenüber den angrenzenden Bereichen des stabförmigen Körpers (91) in radialer Richtung gesehen vergrößert ist.

18. Harnröhrenverschluß nach einem der Ansprüche 15 bis 17, dadurch gekennzeichnet, daß an dem Stabilisator (98) ein Anschlag (101) vorgesehen ist, der im Durchmesser größer als das Lumen (97) ist.
